# EUROPEAN PATENT APPLICATION

(11) **EP 4 696 770 A1**
(43) Date of publication of application: **18.02.2026**
(21) Application number: 24787996.8
(22) Date of filing: 07.04.2024
(51) Int. Cl.: C12N 1/20, A61K 35/747, A61P 25/24, A61P 25/22

(54) **LEVILACTOBACILLUS BREVIS AND USE THEREOF**

(30) Priority: 12.04.2023 CN 202310388560; 02.04.2024 CN 202410395122
(71) Applicant: IBIOME Biotechnology Co., Ltd., Hefei, Anhui 230601 (CN)
(72) Inventor: ZHU, Shu, Hefei, Anhui 230601 (CN); HU, Ji, Hefei, Anhui 230601 (CN); WEN, Wen, Hefei, Anhui 230601 (CN); TAO, Wanyin, Hefei, Anhui 230601 (CN)
(74) Representative: Santarelli
(86) International application number: PCT/CN2024/086374
(87) International publication number: WO 2024/212893

(57) **Abstract**

Provided is a *Levilactobacillus brevis* ibiome003, and further provided are a pharmaceutical composition, a microbial agent composition and a food composition containing the strain. It is found that the microbial agent can effectively prevent, treat and relieve depression caused by various reasons.

## Description

### Technical Field

The invention relates to the technical field of biology, specifically to a *Levilactobacillus brevis* and application thereof.

### Background of the Invention

Brain-gut axis is composed of complex reflection network including central nervous system (CNS), neuroendocrine system, neuroimmune system, the sympathetic nerve and parasympathetic nerve of the autonomous nervous system (ANS), enteric nervous system (ENS), and a composed of gut microbiota. The signal of the brain can influence the motility, perception and secretion of the intestinal tract through the bidirectional regulating communication network, and the intestinal flora also can influence the brain function. Research has shown that the intestinal microbial composition of patients with depression is altered, and that the intestinal microbiota is interconnected with both quality of life and depression, germ-free mice have a depressive phenotype relative to SPF mice, More and more studies have shown that intestinal microorganisms are closely associated with depression and stress-related disorders, and that intestinal bacteria provide new targets for the treatment of depression.

At present, the most common antidepressant medications mainly act on monoamine neurotransmitter by increasing content in synaptic cleft, which can improve mood and improve power to improve depressive symptoms. Antidepressants including monoamine oxidase inhibitors (MAOls), norepinephrine and dopamine reuptake inhibitors (NDRI), selective serotonin reuptake inhibitors (SSRIs), serotonin and norepinephrine reuptake inhibitors (SNRI), serotonin antagonists and re-uptake inhibitors (SARI) and tricyclic (TCAs) and tetracyclic antidepressants, except for obvious side effects including headache and dizziness, gastrointestinal discomfort, sexual dysfunction and addiction, there is a great difference in clinical remission among patients, and about 30 % of patients cannot achieve remission, and even the same patient cannot maintain stable medical effect at different times.

At present, a few patents and documents have made reports on the improvement of depression by probiotics, the patent CN110157647B of *Lactobacillus brevis* (*Levilactobacillus brevis* used as the name) has excellent GABA production ability, so as to relieve the anxiety of mice, improve sleep, shows outstanding performance in open field test, elevated plus maze and prolonging sleep time in Sodium pentobarbital, but it is not clear about the situation of relieving depression. Patent TW1750788B discloses a *Lactobacillus brevis* GKJOY and a composition that also produces GABA promotes neural function by increasing dopamine and/or serotonin content in brain tissue without disclosing physiological functional data in mice and humans.

### Summary of the Invention

### Technical Solution

The invention relates to a *Levilactobacillus brevis (Levilactobacillus brevis)* ibiome003, the strain is preserved in China Centre for Type Culture Collection, the address is Wuhan University China Typical Culture Preservation Centre in Wuchang District of Wuhan City, Hubei Province, the preservation date is March 21, 2023, and the preservation number is CCTCC NO: M 2023387. The invention further claims a mixture of the *Levilactobacillus brevis* according to any proportion.

The invention also relates to a medicine, comprising the *Levilactobacillus brevis* and pharmaceutically acceptable excipient, carrier, auxiliary material and medium.

Examples of excipients, carriers, adjuvants, vehicles include, but are not limited to antitackiness agents, binders, coatings, compression aids, disintegrants, dyes, lubricants, emulsifiers, fillers (diluents), film-forming agents or coatings, flavorings, fragrances, glidants (flow enhancers), lubricants, adsorbents, suspending or dispersing agent or sweetening agent. Exemplary excipients, carriers, adjuvants, vehicles include, but are not limited to: butylated hydroxytoluene (BHT), calcium carbonate, calcium phosphate (monohydrogen), calcium stearate, cross-linked carboxymethylcellulose, cross-linked polyvinylpyrrolidone, citric acid, cross-linked povidone, cysteine, ethyl cellulose, gelatin, hydroxypropyl cellulose, hydroxypropyl methylcellulose, lactose, magnesium stearate, maltitol, mannitol, methionine, methyl cellulose, methyl p-hydroxybenzoate, microcrystalline cellulose, polyethylene glycol, povidone, pregelatinized starch, propyl p-hydroxybenzoate, retinol palmitate, shellac, silica, sodium carboxymethyl cellulose, sodium citrate, sodium glycolate starch, sorbitol, starch (corn), stearic acid, stearic acid, sucrose, talc, titanium dioxide, vitamin A, vitamin E, vitamin C and xylitol.

The medicine can be prepared in the form of injection preparation or oral preparation. The injection preparation comprises liquid injection, powder for injection and tablet for injection according to the physical state classification; according to the classification of the injection part, comprising intradermal injection, subcutaneous injection, muscle injection, intravenous injection and spinal cavity injection; Preferably, the solvent of the injection preparation comprises injection water or physiological saline.

Formulations for oral use include tablets containing an active ingredient in admixture with a non-toxic pharmaceutically acceptable excipient. These excipients may be, for example, inert diluents or fillers (e.g., sucrose, sorbitol, sugar, mannitol, microcrystalline cellulose, starch including potato starch, calcium carbonate, sodium chloride, lactose, calcium phosphate, calcium sulphate or sodium phosphate); granulating agents and disintegrants (e.g., cellulose derivatives including microcrystalline cellulose, starch including potato starch, croscarmellose sodium, alginate or alginic acid); binders (e.g., sucrose, glucose, sorbitol, gum arabic, alginic acid, sodium alginate, gelatin, starch, pregelatinized starch, microcrystalline cellulose, magnesium aluminum silicate, sodium carboxymethylcellulose, methylcellulose, hydroxypropyl methylcellulose, ethylcellulose, polyvinylpyrrolidone or polyethylene glycol); and lubricant, glidant and anti-sticking agent (such as magnesium stearate, zinc stearate, stearic acid, silicon dioxide, hydrogenated vegetable oil or talcum). The formulation for oral use may also be in the form of a chewable tablet, or in the form of a hard gelatin capsule, wherein the active ingredient is combined with an inert solid diluent (e.g., potato starch, lactose, microcrystalline cellulose, calcium carbonate, Calcium phosphate or kaolin), or in the form of a soft gelatin capsule, wherein the active ingredient is mixed with water or an oil medium (e.g., peanut oil, liquid paraffin, or olive oil). The powders, granules and pills may be prepared in conventional manner using the ingredients mentioned above under tablets or capsules, for example, a mixer, a fluidized bed device or a spray drying device.

Other pharmaceutically acceptable excipients for oral preparations include, but are not limited to, colorants, flavoring agents, plasticizers, humectants, and buffers. The formulation for oral use may also be in the form of a chewable tablet, or in the form of a hard gelatin capsule, wherein the active ingredient is combined with an inert solid diluent (e.g., potato starch, lactose, microcrystalline cellulose, calcium carbonate, Calcium phosphate or kaolin), or in the form of a soft gelatin capsule, wherein the active ingredient is mixed with water or an oil medium (e.g., peanut oil, liquid paraffin, or olive oil). The powders, granules and pills may be prepared in conventional manner using the ingredients mentioned above under tablets or capsules, for example, a mixer, a fluidized bed device or a spray drying device.

In some embodiments, administration includes intramuscular, intravenous (e.g., in the form of a sterile solution and in a solvent system suitable for intravenous use), intradermal, intraarterial, intraperitoneal, intralesional, intracranial, intraarticular, intraprostatic, intrapleural, intratracheal, intranasal, intravitreal, intravaginal, intrarectal, transsurface, intratumoral, intraperitoneal, subcutaneous, subconjunctival, intracapsular, transmucosal, intrapericardial, intraumbilical, intraocular, oral (e.g., tablet, capsule, caplet, caplet or syrup), transsurface (e.g., in the form of a cream, gel, lotion or ointment), topical, by inhalation, The drugs described herein are administered by injection or by infusion (e.g., by continuous infusion in the form of a cream or lipid composition, directly soaking local perfusion of target cells, placing tubes, lavage).

The present invention also relates to a pharmaceutical composition, comprising the above-mentioned *Levilactobacillus brevis* and a combination drug, wherein the combination drug is other substances having synergistic effect with the *Levilactobacillus brevis,* including but not limited to other bacteria, such as: *Lactobacillus casei* (*L. casei*), *Lactobacillus acidophilus* (*L. acidophilus), Lactobacillus plantarum* (*L. plant*), *Lactobacillus helveticus* (*L. helveticus*)*, Lactobacillus fermentum* (*L. fermentum), Lactobacillus curvatus* (*L. curvatus*)*, Lactobacillus sakei* (*L. Sake*)*, Lactobacillus bulgaricus* (*L. bulgaricus*)*, Streptococcus thermophilus, Streptococcus lactis, Lactococcus cremoris, Bifidobacterium longum, Bifidobacterium breve, Bifidobacterium bifidum, Bifidobacterium infantis, Bifidobacterium adolescentis,* et al.; or other pharmaceutically active ingredients, such as 5-hydroxytryptamine reuptake inhibitor (SSRI), 5-hydroxytryptamine and norepinephrine reuptake inhibitor (SNRI), tricyclic antidepressant (TAC), monoamine oxidase inhibitor (MAOI), tetracyclic antidepressant (TeCA), noradrenaline and specific 5-hydroxytryptamine antidepressant (NaSSA) and so on.

Dosage: The dosage of the medicine/medicine composition is 10⁷ to 10¹² CFU.

Period of administration: Adjusted to achieve therapeutic effect including, but not limited to, 1-3 times a day, 3-7 days a week, and the like, as well as related to the concentration of the specific formulation.

### Fermentation agents, and functional microbial inoculum

A fermentation composition or fermentation agent or functional microbial inoculum comprising the bacterial suspension prepared by mentioned *Levilactobacillus brevis,* or powder or granule obtained by further processing; The fermenting agent further comprises more than one non-antagonistic microbial inoculum selected from more than one of acid-resistant lactobacillus, lactobacillus plantarum and lactobacillus bulgaricus to prepare the composite microbial inoculum.

The effective microbial inoculum concentration, viable count is 10⁷ to 10¹²CFU.

Fermentation agents, or functional microbial inoculum can also be used as functional food and nourishment.

### Nutritional composition

A nutrient composition comprising mentioned *Levilactobacillus brevis,* comprises aforementioned *Levilactobacillus brevis* and food, nutriment, supplement, probiotics or symbiotic bacteria.

The food, including the aforementioned *Levilactobacillus brevis* and the auxiliary substance that enable the functional properties of the food., can be presented in the form including but not limited to the dietary supplement, fermented food and so on.

The dietary supplement comprises the bacterial suspension prepared by the *Levilactobacillus brevis* culture or the powder obtained by further processing, and the dietary supplement is obtained by further adding the nutrient substances such as cellulose, vitamin and mineral substance.

The fermented food comprises dairy product, bean product or fruit and vegetable product. Mentioned dairy product is milk, acid cream or cheese and so on. The bean product is soybean milk, fermented black beans or soybean paste. Mentioned fruit and vegetable products are cucumber, carrot, beet, celery or cabbage products.

The probiotic is a living microorganism which, when provided in a suitable amount, is beneficial to the health of the host organism.

The symbiotic bacteria refer to the food containing the mixture of probiotics and probiotics. They typically comprise probiotic components that facilitate growth and/or metabolic activity, and, in general, probiotic effects such as, but not limited to, *Levilactobacillus brevis* in combination with fructooligosaccharides or galactooligosaccharides.

### Pharmaceutical and therapeutic use

The invention claims a use of *Levilactobacillus brevis* or composition in preparing medicine or fermentation microbial inoculum for treating, relieving, preventing, depression and/or anxiety. The manifestations of the depression include, but are not limited to: a) giving up hope of escape; b) acting in despair; c) inability to escape from a hostile environment; d) loss of pleasure; e) lack of interest in reward stimulus; f) long-term depression, grief, emptiness, and hopeless; g) slow thinking, sluggish reaction, hypomnesis and attention decline; h) reducing the self-evaluation, generating useless feeling, hopeless and non-value feeling; i) feeling of guilty, feeling of illness; j) appearing Hallucinations, auditory hallucinations, visual hallucinations; k) there is no interest in almost all activities, or there is no fun; I) the decline of consciousness activity manifested as slow behavior, passive living, lazy, unwilling to do something, unwilling to contact with the surrounding people, often sitting alone on one side, or lying on the bed all day, living alone behind closed doors, living away from relatives and friends, avoiding social activities; m) nausea, vomiting, palpitation, chest distress, sweating and so on; n) the weight is obviously reduced or increased under the condition of not dieting, and the appetite is reduced or increased; o) insomnia or excessive sleep; p) Frequently restless, often feeling fatigued or energetic, including factors induced by physical environments, chemical substances, and certain foods. Specifically, due to chronic restraint stress (CUMS), or caused by chronic unpredictable mild stress model (CUMS), or caused by learned helplessness (LH) model, or caused by social defeat (SD) model, or caused by amino acid food (AAD), or cortisone (Cort), or genetic (family history or gene association, the risk of the patient's first-level relative (parents, children, parents, brothers and sisters) suffering from depression is about 2-10 times more than that of ordinary people, the genetic degree is 31%-42%), medicine (suffering from other physiological diseases, trauma or administration of certain drugs, such as: Parkinsonism, epilepsy, digestive system diseases, hypothyroidism and other diseases may lead to depression), psychology (including how patients view others and themselves, how to establish contact with the outside world; Several psychological characteristics that may cause depression include: The environment (which refers to events or phenomena that may lead to depression by the outside world) is weak in anti-pressure ability, helpless, angry, desperate, self-confidence and so on, and the more common social factors include: family problems, such as marital conflicts; living pressure, such as unemployment; traumatic events, such as traffic accidents, death of relatives; the psychological trauma of childhood, such as neglect, abuse, parents died at an early age, and so on; In addition, cultural background factors may, to a certain extent, lead to depression.factors, but not limited to these factors.

The *Levilactobacillus brevis* is expected to be used in other nervous system diseases, such as: depression, anxiety disorder, autism, infantile autism, schizophrenia, obsessive compulsive disorder, phobia, bipolar disorder, personality disorder ratio, mania, delusional disorder, suspected disorder, neurasthenia, mental retardation, dementia, insomnia, psychological and physiological disorders, affective disorders, paranoid disorders, alcoholdependent mental disorder, substance-dependent mental disorder, reactive mental disorder (acute stress response, post-traumatic stress disorder, adaptation disorder, sexual physiological disorder (sexual metamorphosis, sexuality, pedophilia, homosexuality and so on), personality disorder anti-social personality disorder, impulse personality disorder, cognitive disorder, The Alzheimer's disease, epilepsy, Parkinson's disease and so on have the same preventing or relieving effect.

### Beneficial effects

The invention claims a preparation method and application of *Levilactobacillus brevis* for relieving depressive symptom of mouse. The *Levilactobacillus brevis* is obtained by separating from the intestinal tract of healthy human, the source is safe and controllable, it is easy to culture in vitro, the colonization efficiency in intestinal tract is high, and it can obviously improve depressive behavior in different depressive models, it can be used for preparing food with antidepressant function. The healthcare product and medicine provide new idea for clinical intervention and control depression and other related mental diseases, and have good and wide application prospect. And *Levilactobacillus brevis* widely exist in fermented food and human intestine, the invention also proves that the strain has no side effect on weight, internal organs, liver and intestinal tissue and has high safety; it also has good tolerance in the simulated gastric juice of pH 3.0 and the simulated intestinal juice of 0.3% bile salt, which shows that it has good acid resistance and bile salt resistance, and that is more beneficial for the drug effect after the patent medicine.

### Biological preservation specification

The *Levilactobacillus brevis (Levilactobacillus brevis)* ibiome003, the preservation date was March 21, 2023, the preservation place is China Centre for Type Culture Collection, the address is China Centre for Type Culture Collection, Wuhan University, Bayi Road, Wuchang District, Wuhan City, Hubei Province, the preservation number is CCTCC NO: M 2023387.

### Brief Description of the Drawings

FIG. 1 is a single colony photograph of *Levilactobacillus brevis* in Example 2;
FIG. 2 shows the coating microscopic examination of the colony of *Levilactobacillus brevis* in Example 2 under gram staining at 40X;
FIG. 3 shows the colonization ability testing of *Levilactobacillus brevis* in Example 3, and the numbers marked between the groups represent the P value between the two;
FIG. 4 shows the effect of the colonization of *Levilactobacillus brevis* 1 (ibiome003) in the depression model induced by CUMS in Example 4 on mouse forced swimming (A), suspension tail (B) and sugar water preference (C); and the numbers marked between the groups represent the P value between the two;
FIG. 5 is a trend graph of weight change of mice in Example 5;
FIG. 6 shows the effect of the administration of *Levilactobacillus brevis* by intraperitoneal injection on the morphological change of the mouse liver;
FIG. 7 shows the effect of the administration of *Levilactobacillus brevis* by intraperitoneal injection on the morphological change of the mouse intestinal tract;
FIG. 8 shows the effect of the *Levilactobacillus brevis* colonization under the depression model induced by AAD in Example 6 on the forced swimming (A), the suspension (B) and the sugar water preference (C) of mice; and the numbers marked between the groups represent the P value between the two;
FIG. 9 shows the effect of the colonization of *Levilactobacillus brevis* 1 (ibiome003) on serotonin (A), acetylcholine (B) and dopamine (C) in the brain of mice under the depression model induced by AAD in Example 7;
FIG. 10 shows the effect of Cort induced depression model in Example 8 on mouse forced swimming (A), suspension (B) and sugar water preference (C) by *Levilactobacillus brevis* colonization, and the numbers marked between the groups represent the P value between the two.
FIG. 11 shows the effect of multiple *Levilactobacillus brevis* colonization under the depression model induced by CUMS in Example 9 on mouse forced swimming (A) and tail suspension (B), and the number labeled between groups in the figure represents the P value between the two.

### Detailed Description of Embodiments

### Example 1 Separation and gene detection of Levilactobacillus brevis

The separation of *Levilactobacillus brevis* from the intestinal tract of healthy human:
Volunteer faecal samples (the employee gift from IBIOME Biotechnology Co., Ltd.) were stored with 20 % glycerol phosphate buffer, diluted to 10-5,10-6,10-7 times in gradient, respectively. 100 µL of each concentration was applied to the culture medium of the GAM broth (Solarbio LA4450) and MRS broth. Selecting monoclonal to the corresponding liquid culture medium, strain genome DNA analyze through 16sRNA universal primer (upstream primer sequence 27F: AGAGTTTG ATCCTGGCTCAG (SEQ ID NO. 4), downstream primer sequence 1492R: GGTTA CCTTGTTACGACTT (SEQ ID NO. 5)); The amplification procedure is as follows: (1) 95 degrees centigrade for 7 min; (2) 95 degrees centigrade, 15 s; (3) 65 degrees centigrade, 15 s; (4) 72 degrees centigrade, 30 s; (5) GO TO step 2, 9x; (6) 95 degrees centigrade, 15 s; (7) 55 degrees centigrade, 15 s; (8) 72 degrees centigrade, 30 s, (9). GOTO step 6, 32x; (10) 72 degrees centigrade, 5 min; (11). 12 degrees centigrade, 30 min. Using glycerol to store at -80 degrees centigrade. The amplification product is sent to sequencing, universal biological company sequencing 16s rDNA sequence compare determining species, 16s rDNA the sequencing sequence as follows:

GGCTGACTCCCGAAGGTTATCTCACCGGCTTTGGGTGTTACAAACTCTCATGGT
GTGACGGGCGGTGTGTACAAGGCCCGGGAACGTATTCACCGCGGCATGCTGATCCGCGATT
ACTAGCGATTCCAACTTCATGTAGGCGAGTTGCAGCCTACAATCCGAACTGAGAACGGCTT

TAAGATTAGCTTAGCCTCACGACTTCGCAACTCGTTGTACCGTCCATTGTAGCACGTGTGTAG
CCCAGGTCATAAGGGGCATGATGATTTGACGTCATCCCCACCTTCCTCCGGTTTGTCACCGG
CAGTCTCACCAGAGTGCCCAACTGAATGCTGGCAACTGATAATAAGGGTTGCGCTCGTTG
CGGGACTTAACCCAACATCTCACGACACGAGCTGACG
ACAACCATGCACCTGTCATTCTGTCCCCGAAGGGAACGTCTTATCTCTAAGATTGGCAGAAGA
TGTCAAGACCTGGTAAGGTTCTTCGCGTAGCTTCGAATTAAACCACATGCTCCACCGCTTGTG
CGGGCCCCCGTCAATTCCTTTGAGTTTCAACCTTGCGGTCGTACTCCCCAGGCGGAGTGCTT
AATGCGTTAGCTGCAGCACTGAAGGGCGGAAACCCTCCAACACTTAGCACTCATCGTTTAC
GGCATGGACTACCAGGGTATCTAATCCTGTTCGCTACCCATGCTTTCGAGCCTCAGCGTCA
GTTACAGACTAGACAGCCGCCTTCGCCACTGGTGTTCTTCCATATATCTACGCATTCCACC
GCTACACATGGAGTTCCACTGTCCTCTTCTGCACTCAAGTCTCCCAGTTTCCGATGCACTTCT
CCGGTTAAGCCGAAGGCTTTCACATCAGACTTAAAAAACCGCCTGCGCTTTACGCCCAA
TAAATCCGGACAACGCTTGCCACCTACGTATTACCGCGGCTGCTGGCACGTAGTTAGCCG
TGGCTTTCTGGTTAAATACCGTCAACCCTTGAACAGTTACTCTCAAAGGTGTTCTTCTTTAA
CAACAGAGTTTTACGAGCCGAAACCCTTCTTCACTCACGCGGCATTGCTCCATCAGACTTTCG
TCCATTGTGGAAGATTCCCTACTGCTGCCTCCCGTAGGAGTTTGGGCCGTGTCTCAGTCCCA
ATGTGGCCGATTACCCTCTCAGGTCGGCTACGTATCATCGTCTTGGTGGGCCTTTACCTCA
CCAACTAACTAATACGCCGCGGGATCATCCAGAAGTGATAGCCGAAGCCACCTTTCAAACA
AAATCCATGCGGATTTTGTTGTTATACGGTA
TTAGCACCTGTTTCCAAGTGTTATCCCCTGCTTCTGGGCAGATTCCCCACGTGTTACTCAC
CAGTTCGCCACTCGCTTCATTGTTGAAATCAGTGCAAGC
ACGTCATTCAACGGAAGCTCGTTCGACTGC (SEQ ID NO.1)

### Embodiment 2 Identification of Levilactobacillus brevis

### 2.1 Photographs of individual colonies

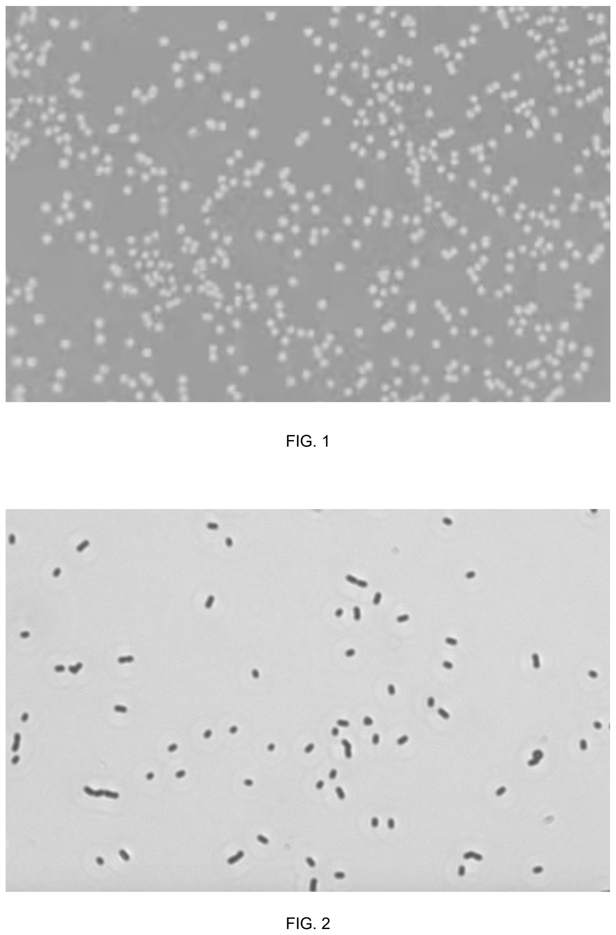

The *Levilactobacillus brevis* was cultured on MRS culture medium for 48 h and then photographed. The single colony was shown in FIG. 1, and the colony was circular, smooth, regular in edge, white, and wet in surface.

### 2.2 Smear microscopic examination

The *Levilactobacillus brevis* was subjected to smear microscopic examination at 40X using a microscope to obtain a microscopic examination pattern as shown in FIG. 2. From the picture, it can be seen that the *Levilactobacillus brevis* is gram-positive, in the form of short rod and without spore.

### 2.3 sugar alcohol fermentation biochemical reaction detection

Taking 300 µ L of frozen *Levilactobacillus brevis* to 1 mL MRS culture medium to resuscitate the strain, taking the *Levilactobacillus brevis* to purify in MRS solid culture medium lineation, selecting single colony to inoculate in 1 mL MRS liquid culture medium to culture for 24h, then taking the liquid cultured strain, through gradient dilution, coating in MRS solid culture medium, culturing for 72h. Then respectively performing sugar alcohol fermentation biochemical reaction detection.

The basic characteristics of the strain were verified and reacted: Detection of sugar alcohol fermentation biochemical reaction

The single bacterial colony was selected by a sterilized gun head to a commercial ampoule bottle for biochemical detection of bacteria (purchased from Qingdao Hopebio technology, Co., Ltd, the number of which is shown in table), inoculated and then placed at 37 °C for anaerobic culture for 48 h, and the detection result was determined according to the specification of the kit, as shown in Table 1:

**Table 1**

| [Table 1_sm-0001] | | | |
|---|---|---|---|
| Sequence number | Cargo number | Name | Results of the assay (positive: +; negative: -) |
| 1 | GB007 | Mannitol fermentation tube | - |
| 2 | GB014 | Salicin fermentation tube | - |
| 3 | GB054 | D-ribose | + |
| 4 | GB055 | D-arabinose | - |
| 5 | GB056 | Lactose | - |
| 6 | GB057 | cellobiose | - |
| 7 | GB060 | Sorbitol | - |
| 8 | GB062 | Gluconate | + |
| 9 | GB102-1 | Trehalose | - |
| 10 | GB104-1 | mebiose | + |
| 11 | GB112 | L-rhamnose | - |
| 12 | GB177 | D-mannose | - |
| 13 | GB178 | D-maltose | + |
| 14 | GB188 | L-arabinose | + |
| 15 | GB193 | D-xylose | + |
| 16 | GB195 | D-sucrose | - |
| 17 | GB196 | Eiyangling | + |
| 18 | GB199 | D-glucose | + |
| 19 | GB200 | D-galactose | + |
| 20 | GB202 | amygdalin | - |
| 21 | GB203 | D-fructose | + |

The detection result proves that: The detected strain is confirmed to be *Levilactobacillus brevis* (*Levilactobacillus brevis*)*.* The strain is named as *Levilactobacillus brevis* (*Levilactobacillus brevis*) ibiome003, preserved in China Centre for Type Culture Collection, the preservation address is: Wuhan University China Center for Type Culture Collection; the preservation date is: on March 21, 2023, and the preservation number is CCTCC NO: M 2023387.

### Example 3 Effect of Levilactobacillus brevis 1 (ibiome003) colonization in the intestinal tract of mice

10 SPF-grade C57/B6j male mice of 6-8 weeks of age were randomly divided into two groups, one group being normal mice + physiological saline group (5 mice), and the other group being normal mice *+ Levilactobacillus brevis* group 1 (5 mice). MRS culture medium cultivating *Levilactobacillus brevis,* 3000rpm, centrifuging for 10min, IxPBS re-suspending to OD1.0 (600nm), by means of gastric lavage, 200 µL of *Levilactobacillus brevis* ibiome003 suspension (10⁷CFU) was administered to 1 group of mice of *Levilactobacillus brevis,* and 200 µL of physiological saline was administered to the group of mice of physiological saline, gastric lavage once every two days, continuous gastric lavage 2w, and detecting the colonized ability of the *Levilactobacillus brevis 1.*

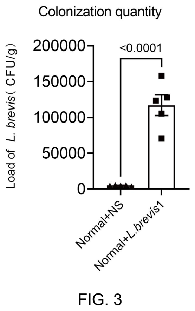

Specifically, mouse faeces are collected in a sterile Ep tube, weighed, and the faeces weight is counted. The DNA in the feces was extracted with an organic solvent (20 % SDS, phenol chloroform, white grinding beads). QPCR quantitative analysis of the load of the strain in mouse excrement by 16s rDNA primer (upstream primer Levil-FAAACAA CTGTATTCCCCA, Levil-RCAACTAATAGGATCACCCCC; internal reference primer, upstream primer 27F: AGAGTTTG ATCCTGGCTCAG, downstream primer 1492R: GGTTA CCTTGTTACGACTT, calculating formula POWER (2, - ΔC) * 100 %, wherein, the Ct target gene-Ct internal reference gene = Δ C), and the result is shown in FIG. 3, the *Levilactobacillus brevis 1* has strong ability of colonization in the intestinal tract of mice, indicating that it may be beneficial for better function.

The QPCR procedure is as follows: (1) 95 degrees centigrade for 2 min; (2) 95 degrees centigrade for 5 s; (3) 60 degrees centigrade, 30 s; (4) GOTO step 2, 40x; (5) 95 degrees centigrade, 10s; (6) 65 degrees centigrade, 5 s; (7)95 degrees centigrade. for 5 min.

### Example 4 Levilactobacillus brevis 1 (ibiome003) colonization alleviates mouse depressive phenotype caused by chronic unpredictable stress (CUMS)

Twenty 6-8 weeks old SPF grade C57/B6j male mice (GemPharmatech corporation) are randomly divided into four groups, one group is normal mouse + physiological saline group (5 mice), one group is normal mouse + *Levilactobacillus brevis* 1 group (5 mice), one group is CUMS mouse + physiological saline group (5), one group is CUMS mouse *+ Levilactobacillus brevis* 1 group (5). After adapting to the environment, from day 0, the group of CUMS mice received chronic, unpredictable mild stimulation at 21d time, simulating the chronic low-intensity stress received in human daily life. Each day a stimulus is selected from the daytime and night to treat the mice randomly, continuously and not repeated, so that the mice cannot predict the appearance of the stimulus. After the continuous modeling 21d, the depression modeling was confirmed to be successful by forced swimming, tail suspension, and sugar water preference experiments. Mouse depression caused by chronic unpredictable stress is depression caused by physical (environmental) factors.

MRS culture medium culture *Levilactobacillus brevis 1,* 3000rpm, centrifuging for 10min, IxPBS re-suspending to OD1.0 (600nm), by means of gastric lavage, 200 µL of *Levilactobacillus brevis* ibiome003 suspension (10⁷ CFU) was administered to 1 group of mice of *Levilactobacillus brevis,* and 200 µL of physiological saline was administered to the group of mice of physiological saline, gastric gavage once every two days, continuous gavage 2w, The mice are tested in forced swimming and tail suspension experiments, and the immobile time of each group of mice in forced swimming and tail suspension experiments is compared, and the specific experiment method is as follows.

The forced swimming experimental instrument is a transparent 20 * 30 cm round barrel filled with purified water, the diameter is 20 cm, and the water depth is 15 cm. the temperature of the pure water is 23-25 degrees centigrade. The front of the instrument is provided with a camera which is parallel to the water surface. After the mice were placed in water, the active video was collected in water for 6 min. The immobile time of the mouse was analyzed in the latest 4 min. The immobile behavior is defined as the behavior of mice floating, not struggling, or depending only on occasional oscillation to maintain the floating behavior, the longer the quiescent time is, the more serious the characterization of depressive conditions. The immobile time in the forced swimming experiment reacts to give up the hope of escaping, that is, the behavior is desperate.

The tail of the mouse was suspended in a position where they were unable to escape or grasp the nearby surface with an adhesive tape for the tail suspension experiment. A camera was installed in front of the mouse to record the active video of the mouse suspended for 6 min and the immobile time of the mouse within latest 4 min after the video was analyzed. This test is based on the fact that animals are subject to short-term unavoidable pressure and develop an inanimate posture. The more severe the depressive phenotype, the less time they try to escape. The suspension tail experiment is to induce the generation of depressive desperate behaviour through the inability to overcome abnormal body position, that is, the desperate behaviour caused by the inability to escape the evil environment.

The sugar-water preference experiment is used for evaluating the mouse sugar-water preference, which can reflect the mouse pleasure (pleasant feeling) loss degree. In the whole animal experiment, two sugar-water preference experiments are carried out. Before modeling, the sucrose preference coefficient of all mice is measured by the sugar-water preference experiment so as to ensure the consistent state of the tested mice. After the experiment is finished, the sugar water preference experiment is formally carried out, comprising an adaptive training part and a testing part. In training, two bottles of 1 % (w/v) sucrose solution were placed in each cage in the first 24 h, and then one bottle was replaced with pure water in the next 24 h. After the adaptation was completed, mice were fasted and limited to water consuming for 24 h, and then the preference coefficient of the sugar water was measured. In the test, mouse can only select two bottles weighed beforehand, one bottle is 1 % (w/v) sucrose solution, the other bottle is pure water, fasting, after 24h, taking two bottles and weighing, recording mouse total liquid consumption, sugar water consumption and pure water consumption. the calculation mode of the sugar water preference coefficient is as follows: The sugar water preference coefficient (%) = sugar water consumption/(sugar water consumption + pure water consumption) × 100 %. The sugar-water preference experiment can reflect the loss of pleasure (happy feeling), that is, the individual cannot experience happiness from the reward or happy activity; or a form of expression that lacks interest in encouraging stimulation, i.e., affective disorder/depression.

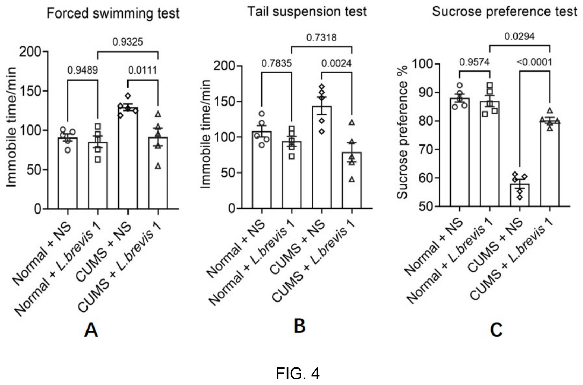

The results are shown in FIG. 4, in the forced swimming and tail suspension experiments (FIGs. 4A and 4B), the immobile time of the group of CUMS mice + *Levilactobacillus brevis 1* is significantly lower than that of the group of CUMS mice + physiological saline, which is substantially equivalent to that of the normal group of mice. It is shown that *Levilactobacillus brevis 1* can significantly alleviate the depressive behavior of mice caused by CUMS. In the sugar-water preference experiment (FIG. 4C), the sugar-water preference degree of the group of CUMS mice *+ Levilactobacillus brevis* 1 is significantly higher than that of the group of CUMS mice + physiological saline (P<0.01), which is substantially equivalent to that of the normal mouse group. It is shown that the *Levilactobacillus brevis 1* can obviously recover the mouse anhedonia -loss-like depressive behaviour caused by CUMS.

### Example 5 Effect of Levilactobacillus brevis 1 (ibiome003) on mice

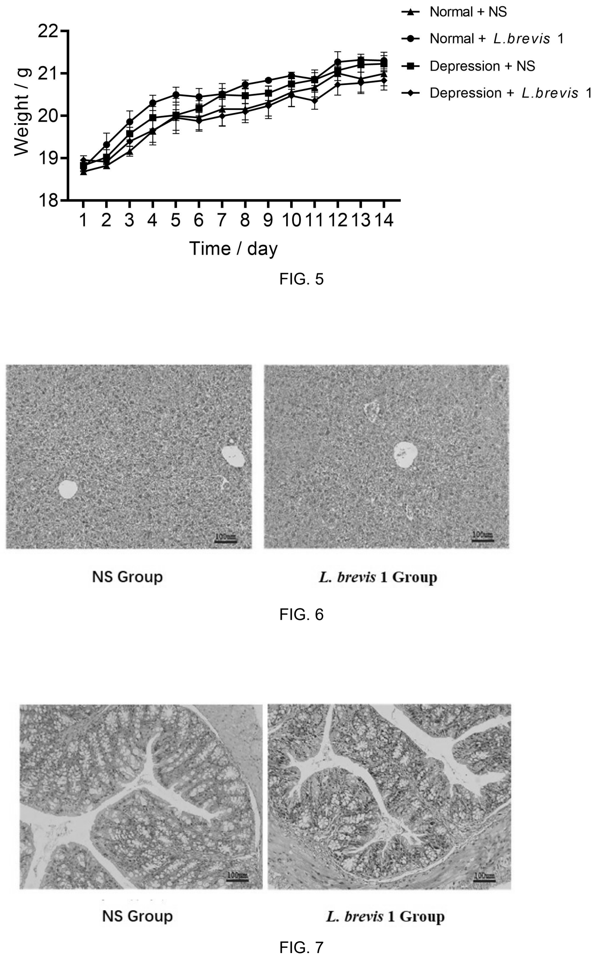

The weight change of the mice in Example 4 was monitored, and the weight data of the mice were recorded daily from the beginning of the feeding of *Levilactobacillus brevis 1,* and the weight curve was plotted. The results are shown in FIG. 5, and the experimental results show that there is no obvious difference between the weight changes of the four groups of mice, which shows that *Levilactobacillus brevis 1* does not affect the weight growth of the mice and has good safety. The observation result shows that the mice in the group of *Levilactobacillus brevis* have good safty, and there is no adverse reaction during the observation period, which is not obviously different from the mice in the normal group.

After the experiment, the tissue was obtained, and the internal organs of the mice in the group of *Levilactobacillus brevis 1* were healthy and plump, and there was no organ lesion, and there was no adhesion phenomenon between the organs, and the liver and intestinal tissue were subjected to HE staining, as shown in FIG. 6 and FIG. 7, and there was no difference in cell morphology. At the same time, the ALT and AST indexes in the mouse serum are evaluated without any side effect.

### Example 6 Levilactobacillus brevis 1 (ibiome003) colonization alleviate mouse depressive phenotype caused by amino acid food (AAD).

Twenty 6-8 weeks old SPF grade C57/B6j male mice (GemPharmatech corporation) are randomly divided into four groups, one group is normal mouse + physiological saline group (5 mice), one group is normal mouse + short fermenting agent lactobacillus 1 group (5 mice), one group is AAD mouse + physiological saline group (5 mice), one group is AAD mouse + *Levilactobacillus brevis* 1 group (5 mice), after adapting to the environment, from day 0, the normal mouse group is fed with common mouse feed, the AAD mouse group is fed with amino acid food (teluffi, amino acid diet), continuously feeding 4w, through forced swimming, tail suspension, sugar water preference experiment confirming depressive modeling success. Mouse depression caused by amino acid food belongs to depression induced by food.

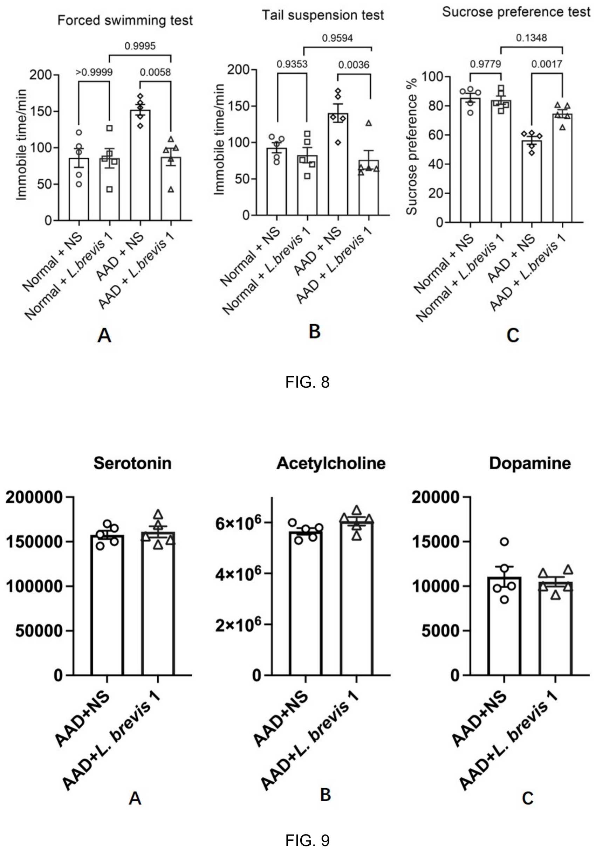

Also by gastric gavage, *Levilactobacillus brevis* 1 (10⁹ CFU) was colonized in the intestinal tract of mice for a period of 2 w. The mice were tested for forced swimming and suspension tail experiments, and the immobile time for each group of mice in the forced swimming and tail suspension experiments was compared. As a result, as shown in FIG. 8A and FIG. 8B, the immobile time of the group of AAD mice *+ Levilactobacillus brevis* 1 is significantly lower than that of the group of AAD mice + physiological saline, which is substantially equivalent to that of normal mice. It is shown that *Levilactobacillus brevis 1* can significantly alleviate the depressive-like behavior of mice caused by amino acid food. The sugar-water preference experiment result is shown in FIG. 8C, the sugar-water preference degree of AAD mouse *+ Levilactobacillus brevis* group 1 is significantly higher than that of AAD mouse + physiological saline group (P < 0.01), which is substantially equivalent to that of normal mouse group, It is shown that *Levilactobacillus brevis 1* can significantly recover mouse-like depressive behavior caused by AAD.

### Example 7 Content Detection of Serotonin, Acetylcholine and Dopamine

The inventor respectively gavage physiological saline and *Levilactobacillus brevis 1* (ibiome003) to the depressed mice caused by amino acid food (AAD) in Example 6, The content of serotonin, and dopamine were tested in brain, as shown in FIG. 9, and the results show that the levels of serotonin, acetylcholine and dopamine in the brain of the depressed mice of *Levilactobacillus brevis* 1 were similar to the levels in the brain of the depressed mice of physiological saline mice, and there was no significant change.

### Example 8 Levilactobacillus brevis 1 (ibiome003) Mice alleviates depressive-like phenotype caused by Cort

Twenty 6-8 weeks old SPF grade C57/B6j male mice (GemPharmatech corporation) are randomly divided into four groups, one group is normal mouse + physiological saline group (5 mice), one group is normal mouse + *Levilactobacillus brevis* 1 group (5 mice), One group was Cort mice + physiological saline group (5 mice), and one group was Cort mice + *Levilactobacillus brevis* group 1 (5 mice). After adapting to the environment, starting from day 0, the drinking water of Cort mouse group (MCE, HY-B1618) is the cortisone solution with final concentration of 25 µ g/mL (pH 7.0-7.4), the normal mouse group receives common drinking water, every 2d is replaced once, continuous modeling 21d. after the modeling is finished, the model is confirmed to be successful through forced swimming, tail suspension and sugar water preference experiment. The mouse depression caused by corticosterone (Cort) belongs to the depression caused by chemical factors such as chemical substances.

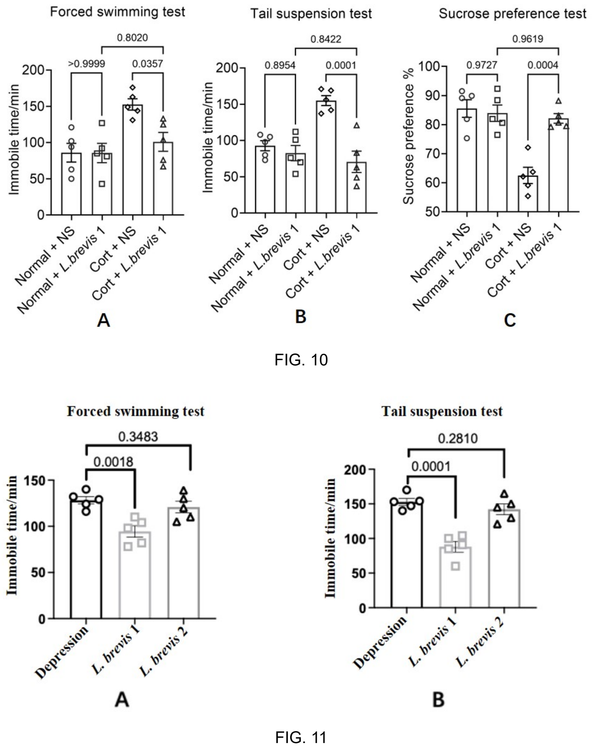

Also by gastric gavage, *Levilactobacillus brevis* 1 (10¹²CFU) was colonized in the intestinal tract of mice for a period of 2 w. The mice were tested for forced swimming and tail suspension experiments, and the immobile time for each group of mice in the forced swimming and tail suspension experiments were compared. As a result, as shown in FIG. 10A and FIG. 10B, the immobile time of the group of Cort mice *+ Levilactobacillus brevis* 1 was significantly lower than that of the group of Cort mice + physiological saline, substantially equivalent to that of normal mice. It is shown that *Levilactobacillus brevis 1* can significantly alleviate the depressive-like behavior of mice caused by corticosterone. The sugar-water preference experiment result is shown in FIG. 10C, the sugar-water preference index of the Cort mouse *+ Levilactobacillus brevis* group 1 is significantly higher than that of the Cort mouse + physiological saline group (P < 0.01), which is substantially equivalent to the normal mouse group. It is shown that *Levilactobacillus brevis 1* can significantly restore Cort-induced mouse-like depressive behavior.

### Example 9 Not All of the Levilactobacillus brevis can alleviate depressive symptoms

We tested several healthy human intestinal source of *Levilactobacillus brevis,* here a reference *Levilactobacillus brevis* 2 as contrast and test. The 6-8 weeks old SPF grade C57/B6j male mice (GemPharmatech corporation) are randomly divided into three groups, one group is depressive group + physiological saline group (5 mice), One group is depressive group + *Levilactobacillus brevis* 1 group (5), one group is depressive group + control *Levilactobacillus brevis* 2 group (5). After adapting to the environment, from day 0, mice received chronic unpredictable mild stimulation at 21 d, simulating the chronic low-strength stress received in human daily life. Each day a stimulus is selected from the daytime and night to treat the mice, randomly and continuously not repeated, so that the mice cannot predict the appearance of the stimulus. After the continuous modeling 21d, the depression model was confirmed to be successful by forced swimming, tail suspension, and sugar water preference experiments. In the same way, in the mouse intestinal tract, *Levilactobacillus brevis* is colonized for 2 w. The mice were tested for forced swimming and tail suspension experiments, and the immobile time for each group of mice in the forced swimming and tail suspension experiments were compared.

As shown in FIG. 11, there is no obvious difference between the immobile time of the mice gavaged control *Levilactobacillus brevis* 2 and control depressive mice in forced swimming and tail suspension test, and the degree of depression has not been alleviated, which proves that not all the *Levilactobacillus brevis* has the function of alleviating depression.

### Example 10 Levilactobacillus brevis 1 (ibiome003) has acid and cholate resistance

### (1) Experimental Grouping

Acid resistance test: A. buffer control group; B. pH 3.0 simulated gastric juice group
Cholate resistance test: A. buffer control group; B. 0.3 % bile salt simulated intestinal fluid group

### (2) Reagent Formulating

pH 3.0 simulated gastric juice: The pepsin 0.25 g was added to 20 ml of water, the pH was adjusted to 3, and the volume was fixed to 25 ml by adding water.
Buffer: 6.8 g of potassium dihydrogen phosphate was dissolved in 500 mL of pure water, and the pH value was adjusted to 6.8 with 0.1 mol/L of sodium hydroxide solution.
Bile salt simulated intestinal fluid: taking bile salt solid according to proportion to prepare 20 ml 0.3% bile salt simulated intestinal liquid.

### (3) Operation

Taking 2 g of *Levilactobacillus brevis* 1 bacteria powder, vortex in 18 mL of sterile physiological saline until completely dissolving, preparing for test liquid.

Taking 1 mL of test solution, respectively adding to 9 mL of buffer solution or pH 3.0 simulated gastric juice to mix uniformly. After standing, sampling, the sampling time point: 0h, 0.5h, 1h, 2h. The 100 µL sample was diluted by 10-fold gradient and then the appropriate gradient was selected, and 100 µL was coated on the surface of the MRS culture medium. Selecting four gradients according to the time change, after coating, culturing at 30 degrees centigrade, observing and recording the colony growth condition.

Taking 2 mL of the test solution, respectively adding 18 mL of buffer solution or 18 mL of 0.3 % cholate simulated intestinal solution, uniformly mixing, standing and then respectively mixing for 0 h, 1.5 h, 3 h, 4.5 h, 6 h, sampling 100 µ L, after 10 times gradient dilution, selecting proper gradient, 100 µL was coated on the surface of MRS culture medium. selecting four gradients according to the time change, after coating, culturing at 30 degrees centigrade, observing and recording the colony growth condition.

### (4) Results

**Table 2 Acid-resistant results statistics of Levilactobacillus brevis 1 bacteria powder**

| Placement time | Viable count (CFU/g) | |
|---|---|---|
| | Buffer | pH3.0 simulated gastric juice |
| 0h | 2.83×10¹¹ | 2.83×10¹¹ |
| 0.5h | / | 2.74×10¹¹ |
| 1h | / | 2.16×10¹¹ |
| 2h | 2.8×10¹¹ | 1.05×10¹¹ |

**Table 3 Statistics on bile salt resistant results of Levilactobacillus brevis 1 bacteria powder**

| Placement time | Viable count (CFU/g) | |
|---|---|---|
| | Buffer | 0.3% bile salt simulated intestinal fluid |
| 0h | 9.33×10¹¹ | 9.70×10¹¹ |
| 1.5h | / | 7.23×10¹¹ |
| 3h | / | 5.70×10¹¹ |
| 4.5h | / | 5.70×10¹¹ |
| 6h | 10.10×10¹¹ | 3.97×10¹¹ |

The result shows that the *Levilactobacillus brevis 1* has good tolerance in the simulated gastric juice solution with pH of 3.0 and the viable count is reduced slowly. The reduction amplitude in the 0.3 % bile salt simulated intestinal solution is not large, which shows that the *Levilactobacillus brevis 1* has good acid resistance and bile salt resistance.

### Industrial practicability

The invention Claims a preparation method and application of *Levilactobacillus brevis* for relieving depressive symptom of mouse. The *Levilactobacillus brevis* is obtained by separating from the intestinal tract of healthy human, the source is safe and controllable, it is easy to culture in vitro, the colonization efficiency in intestinal tract is high, and it can obviously improve depressive behavior in different depressive models, it can be used for preparing food with antidepressant function, The healthcare product and medicine provide new idea for clinical intervention and control depression and other related mental diseases, and have good and wide application prospect. The invention also proves that the strain has no side effect on weight, internal organs, liver and intestinal tissue, and it has high safety; it also have good tolerance in simulated gastric juice with pH of 3.0 and the simulated intestinal juice with pH of 0.3 % of cholate, which shows that it has good acid resistance and cholate resistance, which is more beneficial for the drug effect after the patent medicine, and has industrial practicability.

## Claims

1. A *Levilactobacillus brevis (Levilactobacillus brevis)* ibiome003, **characterized in that**: the *Levilactobacillus brevis* strain is preserved in China Centre for Type Culture Collection, with an address of Wuhan University China Centre for Type Culture Collection, Wuchang District, Wuhan City, Hubei Province, a preservation date of March 21, 2023, and a preservation number of CCTCC NO: M 2023387.

2. A medicine, **characterized in that**, comprising the *Levilactobacillus brevis* (*Levilactobacillus brevis*) ibiome003 according to claim 1, and pharmaceutically acceptable excipients, carriers, adjuvants, and media.

3. The medicine according to claim 2, **characterized in that**: the medicine is powder, suspension, granule, capsule, tablet, pill, oral liquid, injection, or powder for injection.

4. A pharmaceutical composition, **characterized in that**: comprising the *Levilactobacillus brevis* (*Levilactobacillus brevis*) ibiome003 according to claim 1 and a combined medicine, the combined medicine is other medicine which has synergistic effect with the *Levilactobacillus brevis (Levilactobacillus brevis).*

5. A fermentation agent, functional bacterial agent, or nutritional composition, comprising the *Levilactobacillus brevis (Levilactobacillus brevis)* ibiome003 according to claim 1.

6. The fermentation agent, functional bacterial agent, or nutritional composition according to claim 5, **characterized in that**: the nutritional composition is a food, nutritional supplement, dietary supplement, probiotic, or synbiotic.

7. The fermentation agent, functional bacterial agent, or nutritional composition according to claim 6, **characterized in that**: the food includes ordinary food and special food, preferably, the special food includes health foods, formula foods for special medical purposes, and formula foods for infant.

8. Use of the *Levilactobacillus brevis (Levilactobacillus brevis)* ibiome003 according to claim 1, or the medicine according to any one of claims 2-3, or the pharmaceutical composition according to claim 4 in the preparation of a drug for treating, alleviating, or preventing depression and/or anxiety disorders.

9. The use according to claim 8, **characterized in that**, a symptom of the depression comprises but not limited to:
a) giving up hope of escape;
b) acting in despair;
c) inability to escape from a hostile environment;
d) loss of pleasure;
e) lack of interest in rewarding stimulus.

10. The use according to claim 8, **characterized in that**: the depression is caused by physical (environmental) factors, chemical factors, and/or specific foods.
